# FASCICULE DE BREVET EUROPEEN

(11) **EP 0 906 751 B1**
(45) Date de publication et mention de la délivrance du brevet: **20.04.2005**
(21) Numéro de dépôt: 98420169.9
(22) Date de dépôt: 30.09.1998
(51) Int. Cl.: A61F 2/40, A61F 2/38, A61B 17/15, A61B 17/17

(54) **Prothèse destinée à être ancrée dans un os long**
Prothese zum Verankern in einem langen Knochen
Prosthesis to be anchored in a long bone

(30) Priorité: 01.10.1997 FR 9712447
(43) Date de publication de la demande: 07.04.1999
(73) Titulaire: TORNIER SA, 38330 Saint-Ismier (FR)
(72) Inventeur: Tornier, Alain, 38330 Saint-Ismier (FR)
(74) Mandataire: Myon, Gérard Jean-Pierre

(56) Documents cités:
- EP-A- 0 786 238
- EP-A- 0 821 924
- WO-A-97/16129
- FR-A- 2 528 307
- US-A- 3 228 393
- US-A- 4 623 353
- US-A- 4 770 660
- US-A- 5 047 035
- US-A- 5 100 407
- US-A- 5 163 964
- US-A- 5 258 032

## Description

La présente invention concerne une prothèse destinée à être ancrée dans un os long.

De manière habituelle, ce type de prothèses comprend une queue destinée à être insérée dans le canal médullaire de l'os, prolongée par une partie métaphysaire se raccordant à une collerette permettant un appui sur la métaphyse de l'os.

Ce type de prothèses est susceptible d'être implanté notamment à l'extrémité supérieure d'un humérus, d'un fémur ou d'un tibia, par exemple en cas de fracture ou d'affections telles que l'arthrose ou la polyarthrite.

Après insertion de cette prothèse dans l'os, l'ostéosynthèse induit un accrochage intime de la prothèse et de l'os. En effet, la métaphyse de l'os se reconstitue autour de la partie correspondante de la prothèse et forme des protubérances coopérant avec des renfoncements de la prothèse, voire un pont osseux si cette dernière est pourvue d'un trou traversant.

Toutefois, cet accrochage intime et la présence de protubérances ou d'un pont osseux peut se révéler gênante, dans le cas où il est nécessaire de retirer la prothèse, notamment lors d'une opération ultérieure. Il est alors nécessaire de casser la métaphyse reconstituée au moyen d'outils appropriés, tels qu'un ciseau, un ostéostome ou un foret.

Cette opération se révèle toutefois particulièrement délicate. En effet, la présence de la collerette gêne l'accès à la métaphyse de l'os, qui est située au voisinage de la face de cette collerette opposée à celle accessible par le chirurgien. Ce dernier doit donc endommager l'os au niveau des surfaces latérales de la collerette afin d'accéder à la métaphyse. Outre le fait que cette opération est délicate pour le chirurgien, elle induit une dégradation de l'os sur une surface importante de celui-ci.

Afin de résoudre ce problème, une solution connue consiste à prévoir une prothèse dont la collerette est amovible par rapport à la queue. De la sorte, en cas d'opération postérieure ou analogue, le chirurgien ôte la collerette amovible de manière à pouvoir accéder directement à la métaphyse reconstituée. Cependant, cette solution présente tout d'abord un inconvénient économique, lié à la complexité mécanique de la prothèse et au surcoût qui en résulte. De plus, étant donné que cette prothèse est insérée dans un milieu organique, les éléments mécaniques assurant l'encliquetage amovible de la collerette sur la queue sont susceptibles d'être bloqués par des tissus.

L'invention se propose donc de réaliser une prothèse permettant de pallier les inconvénients de l'art antérieur évoqués ci-dessus.

A cet effet, l'invention a pour objet une prothèse selon les revendications indépendantes 1 et 3.

Des caractéristiques avantageuses de l'invention font l'objet des sous-revendications.

L'invention permet de réaliser les objectifs précédemment mentionnés. En effet, la prothèse conforme à l'invention présente une structure simple qui rend son usinage particulièrement simple.

De plus, la présence de cette ouverture permet au chirurgien de pouvoir désolidariser la prothèse de la métaphyse reconstituée de l'os depuis la face de la collerette qui lui est accessible, sans avoir à endommager l'os au niveau de la partie latérale de la collerette.

On entend par outil de découpe de l'os, tout outil approprié, tel que notamment ciseau, ostéotome ou bien foret.

Selon un premier mode de réalisation, l'ouverture de passage de l'outil est constituée par au moins une fente oblongue. Ce mode de réalisation permet l'utilisation d'un outil de découpe dont la face d'attaque est sensiblement rectangulaire, tel que par exemple un ciseau ou un ostéotome.

Cette fente oblongue peut s'étendre radialement jusqu'à un côté de la collerette. Ceci permet au chirurgien de pouvoir bénéficier d'un angle d'attaque incliné. Les termes radial et axial doivent être compris comme relatifs à la collerette.

Selon un second mode de réalisation, l'ouverture de passage de l'outil est constituée par une série d'orifices, notamment circulaires, de faibles dimensions. Ce mode de réalisation permet l'utilisation d'un outil de découpe tel qu'un foret.

Selon un premier mode de réalisation de l'invention, la partie métaphysaire comprend deux branches se raccordant à la collerette au niveau de zones de raccord, et la collerette comprend deux ouvertures disposées de part et d'autre de l'axe de jonction de ces zones de raccord. Dans ce mode de réalisation, ces deux branches définissent entre elles un volume au sein duquel les fragments osseux de la partie métaphysaire initialement éclatés, sont fixés entre eux de manière à former un pont osseux assurant l'ancrage de la prothèse dans l'os. La présence de ces deux ouvertures disposées de part et d'autre de l'axe joignant les zones de raccord de chaque branche, permet donc de désolidariser le volume osseux défini entre ces branches, par rapport au reste de l'os. La prothèse peut ensuite être retirée aisément, le pont osseux restant éventuellement en place entre ces branches.

De manière avantageuse, ces deux ouvertures s'étendent au moins depuis le voisinage de la zone de raccord entre la première branche et la collerette, jusqu'au voisinage de la zone de raccord de la seconde branche et la collerette.

Selon un autre mode de réalisation de l'invention, la partie métaphysaire comprend une branche prolongeant la queue et se raccordant à la collerette au niveau d'une zone de raccord, ainsi qu'un ergot faisant saillie de la collerette à l'extérieur de la branche, à partir d'une zone de raccord, et la collerette comprend deux ouvertures disposées de part et d'autre de l'axe de jonction des zones de raccord de la branche et de l'ergot.

L'invention va être décrite ci-dessous, en référence aux dessins annexés donnés uniquement à titre d'exemples non limitatifs, et dans lesquels :
- la figure 1 est une vue en perspective partielle, illustrant une première prothèse humérale conforme à l'invention ;
- la figure 2 est une coupe suivant la ligne II-II de la figure 1 ;
- la figure 3 est une vue en perspective partielle illustrant une seconde prothèse humérale conforme à l'invention.

Il est utile, avant de procéder à la description de l'objet de l'invention, de définir un certain nombre de conventions. C'est ainsi que les termes inférieur, supérieur, intérieur et extérieur devront être compris comme se référant à des prothèses portées par un patient debout.

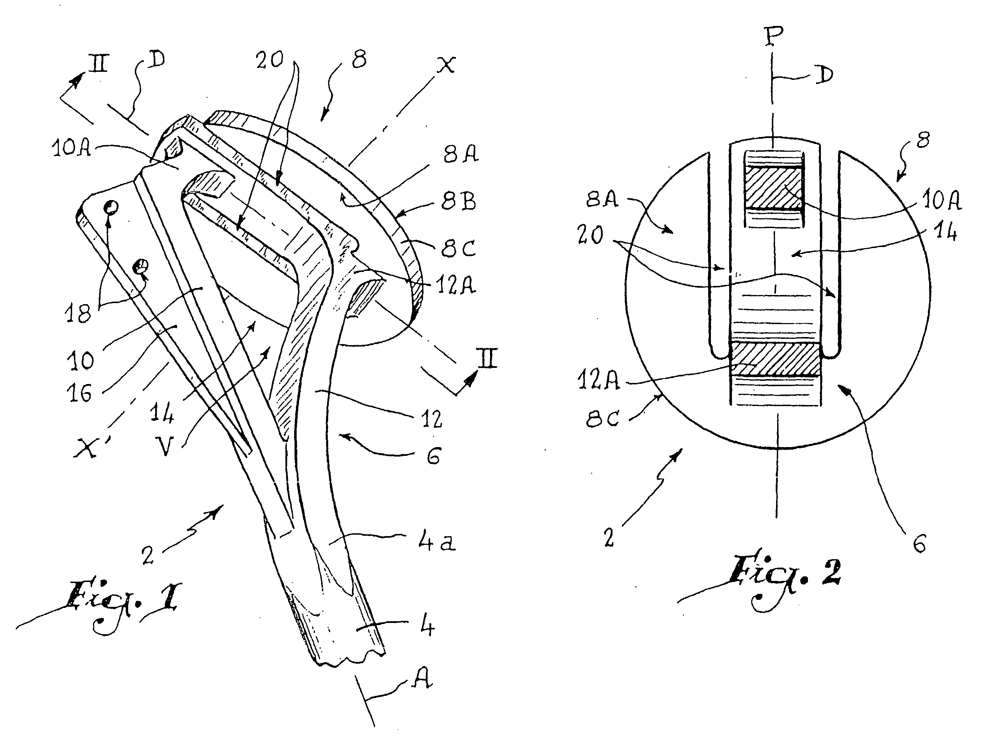

La prothèse représentée à la figure 1 et désignée dans son ensemble par la référence 2, est une prothèse humérale destinée à supporter une calotte hémisphérique non représentée apte à coopérer avec la glène de l'épaule d'un patient.

Cette prothèse comprend une queue 4 de section sensiblement circulaire qui s'engage dans le canal huméral, prolongée par une partie métaphysaire 6 à laquelle est raccordée une collerette 8 destinée à prendre appui sur une métaphyse de l'humérus.

Cette partie métaphysaire 6 comprend une branche extérieure 10 et une branche intérieure 12, qui se raccordent chacune à la face inférieure 8A de la collerette 8, par des zones de raccord respectives 10A, 12A. Ces dernières sont situées sensiblement selon un axe médian de la collerette, à savoir le diamètre D de la collerette disposé dans le même plan P que l'axe A de la queue 4 de la prothèse.

Les zones de raccord 10A, 12A s'étendent uniquement sur une fraction de cet axe médian de manière à définir, à l'extérieur de la zone de raccord 12A de la branche intérieure 12, un évidement 14 constituant un volume libre V de rapprochement et de fusion des fragments d'os de la métaphyse. L'existence de ce volume libre permet la formation d'un pont osseux assurant l'ancrage de cette prothèse dans l'humérus.

La branche extérieure 10 présente une section inférieure à celle de la branche 14 et se prolonge vers l'extérieur par un aileron 16 dont la dimension transversale est inférieure à celle de la branche 10. Cet aileron comprend des perforations 18 permettant le passage de fils de suture.

La collerette 8 est emboîtée, par sa face supérieure 8B inclinée, dans un emboîtement correspondant de la calotte non représentée, destinée à remplacer la tête humérale. Conformément à l'invention, la collerette 8 est pourvue de deux fentes oblongues traversantes 20, disposées de manière sensiblement symétrique de part et d'autre du diamètre D. Chaque fente 20 s'étend radialement à partir de la zone de raccord 12A de la branche intérieure 12, en direction du voisinage de la zone de raccord 10A de la branche extérieure 10, et jusqu'au niveau des parois latérales 8C de la collerette adjacentes à la branche extérieure 10. La largeur de ces fentes 20 est telle qu'elle permette le passage d'un outil de découpe osseuse, tel que par exemple un ostéotome.

Dans le cas où il s'avère nécessaire d'enlever la prothèse 2 de l'os dans lequel elle est implantée, le chirurgien peut découper, grâce à la présence des fentes 20, le pont osseux formé au sein de l'évidement 14, depuis la face supérieure 8B de la collerette 8 qui lui est accessible. Puisque les fentes s'étendent jusqu'aux parois latérales 8C de la collerette, le chirurgien bénéficie d'un angle d'attaque qui peut être dirigé soit parallèlement à l'axe XX' de la collerette, soit perpendiculairement à cet axe, ou qui peut être incliné par rapport à cet axe.

De plus, étant donné que ces fentes 20 sont disposées de part et d'autre du diamètre D de la collerette, le chirurgien est à même de casser le pont osseux en deux endroits, de manière à pouvoir désolidariser la zone osseuse logée dans l'évidement 14, par rapport au reste de l'humérus. Il peut alors enlever aisément la prothèse.

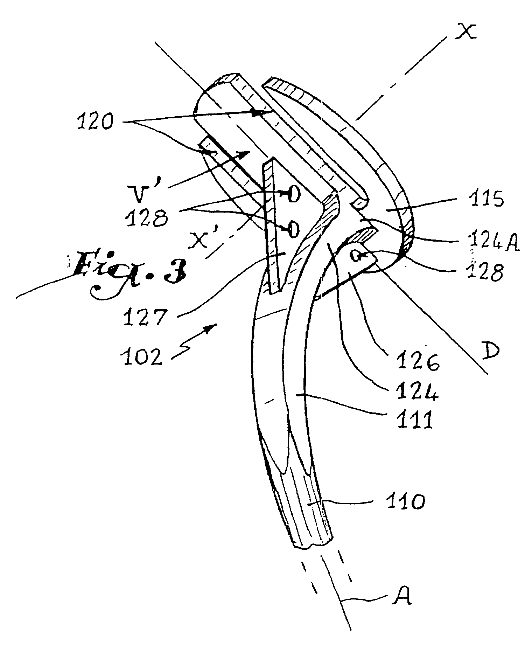

La figure 3 montre un second mode de réalisation de la prothèse conforme à l'invention.

La prothèse 202 représentée à la figure 3 est dépourvue d'aileron. La partie métaphysaire 211 de la prothèse 202 est complétée par un ergot 230 faisant saillie à partir de la portion externe de la collerette, en direction opposée à une calotte non représentée.

La branche 224 et l'ergot 230 se raccordent chacun à la collerette 215 par des zones de raccord respectives 224A et 230A. Ces dernières sont disposées sensiblement sur le diamètre (D) de la collerette, coplanaire avec l'axe (A) de la queue 210 de la prothèse. Ces zones de raccord s'étendent uniquement sur une fraction de ce diamètre de manière à définir, entre la branche 224 et l'ergot 230, un volume libre (V'') de rapprochement et de fusion des fragments d'os de la métaphyse.

L'ergot 230 est prolongé par un aileron 232 s'évasant en direction de la queue 210. Cet aileron 232 présente des dimensions transversales inférieures à celles de l'ergot 230, et se trouve pourvu de perforations 234 permettant le passage de fils de suture.

Etant donné que l'ergot ne s'étend pas jusqu'à la partie proximale 210A de la queue 210, le volume libre (V'') de rapprochement et de fusion des fragments d'os entre eux est ouvert au niveau de cette partie 210A. De plus, la présence de l'ergot 230 assure un appui à l'os spongieux et en empêche l'affaissement.

La collerette 215 est pourvue de fentes 220 qui diffèrent de celles 20 et 120 représentées aux figures précédentes. En effet, elles ne débouchent pas au niveau des parois latérales de la collerette, mais s'étendent uniquement du voisinage de la zone de raccord 224A de la branche 224, jusqu'au voisinage de la zone de raccord 230A de l'ergot 230. Les fentes 220 permettent de séparer de la prothèse la métaphyse de l'os, reconstituée au voisinage des zones de raccord 224A et 230A, en insérant un ciseau parallèlement à l'axe XX' de la collerette 215.

La présente description a été faite uniquement en référence à des prothèses de tibia et d'humérus. L'invention trouve également son application à d'autres os longs, tels que notamment le fémur, ou tout autre emplacement creux dans lequel la présence d'une collerette d'appui entrave l'accès à la tige d'ancrage de la prothèse lorsque son ablation est nécessaire.

## Revendications

1. Prothèse (2) destinée à être ancrée dans un os long, du type comprenant une queue (4) adaptée pour être insérée dans le canal médullaire dudit os, et prolongée par une partie métaphysaire (6) se raccordant, au niveau d'une zone de raccord (10A, 12A) à une collerette (8) d'appui sur la métaphyse dudit os, la partie métaphysaire (6) comprenant une première branche (10) se raccordant à la collerette (8) au niveau d'une première zone de raccord (10A) ainsi qu'une seconde branche (12) se raccordant à la collerette (8) au niveau d'une seconde zone de raccord (12A), ces deux branches (10, 12) définissant un évidement (14) au sein duquel des fragments osseux initialement éclatés de la métaphyse de l'os peuvent être fixés entre eux, ladite collerette comportant deux fentes (20) de passage d'un outil de découpe de la métaphyse reconstituée de l'os, disposées de part et d'autre de l'axe de jonction (D) des zones de raccord (10A, 12A) des deux branches (10, 12).

2. Prothèse selon la revendication 1, **caractérisée en ce que** lesdites deux fentes (20) s'étendent au moins depuis le voisinage de la zone de raccord (10A) entre la première branche (10) et la collerette (8), jusqu'au voisinage de la zone de raccord (12A) entre la seconde branche (12) et la collerette (8).

3. Prothèse (202) destinée à être ancrée dans un os long, du type comprenant une queue (210) adaptée pour être insérée dans le canal médullaire dudit os, et prolongée par une partie métaphysaire (211) se raccordant, au niveau d'une zone de raccord (224A, 230A) à une collerette (215) d'appui sur la métaphyse dudit os, ladite collerette comportant au moins une ouverture (220) de passage d'un outil de découpe de la métaphyse reconstituée de l'os, **caractérisée en ce que** la partie métaphysaire (211) comprend une première branche (224) se raccordant à la collerette (215) au niveau d'une zone de raccord (224A) ainsi qu'un ergot (230) faisant saillie à partir de ladite collerette (215) à l'extérieur de la première branche (224) à partir d'une zone de raccord (230A), et **en ce que** ladite collerette (215) comprend deux ouvertures (220) disposées de part et d'autre de l'axe de jonction (D) des zones de raccord (224A, 230A) de la première branche (224) et dudit ergot (230).

4. Prothèse selon l'une des revendications 1 à 3, **caractérisée en ce que** chaque ouverture est constituée par au moins une fente oblongue (20 ; 220).

5. Prothèse selon la revendication 4, **caractérisée en ce que** chaque fente oblongue (20) s'étend radialement jusqu'à un côté (8C) de la collerette (8).

6. Prothèse selon l'une des revendications 1 à 3, **caractérisée en ce que** chaque ouverture est constituée par une série d'orifices, notamment circulaires, de faibles dimensions.

## Patentansprüche

1. Prothese (2) zur Verankerung in einem langen Knochen, umfassend einen Fortsatz (4), der in den Markkanal des Knochens eingebracht wird und durch einen Metaphysenabschnitt (6) verlängert wird, der in Höhe einer Anheftungszone (10A, 12A) an einem Kragen (8) zur Auflage auf der Metaphyse des Knochens angeheftet ist, wobei der Metaphysenabschnitt (6) einen ersten Schenkel (10), der in Höhe einer ersten Anheftungszone (10A) am Kragen (8) angeheftet ist, sowie einen zweiten Schenkel (12) umfasst, der in Höhe einer zweiten Anheftungszone (12A) am Kragen (8) angeheftet ist, wobei diese beiden Schenkel (10, 12) eine Aussparung (14) bilden, in deren Innerem ursprünglich zersplitterte Knochenfragmente der Metaphyse des Knochens untereinander fixiert werden können, wobei der Kragen zwei Spalte (20) für das Hindurchtreten eines Werkzeugs zum Schneiden der rekonstituierten Metaphyse des Knochens aufweist, die sich auf beiden Seiten der Verbindungsachse (D) der Anheftungszonen (10A, 12A) der beiden Schenkel (10, 12) befinden.

2. Prothese nach Anspruch 1, **dadurch gekennzeichnet, dass** die beiden Spalte (20) sich mindestens von der Nachbarschaft der Anheftungszone (10A) des ersten Schenkels (10) am Kragen (8) bis in die Nachbarschaft der Anheftungszone (12A) des zweiten Schenkels (12) am Kragen (8) erstrecken.

3. Prothese (202) zur Verankerung in einem langen Knochen, umfassend einen Fortsatz (210), der in den Markkanal des Knochens eingebracht wird und durch einen Metaphysenabschnitt (211) verlängert wird, der in Höhe einer Anheftungszone (224A, 230A) an einem Kragen (215) zur Auflage auf der Metaphyse des Knochens angeheftet ist, wobei der Kragen mindestens eine Öffnung (220) für das Hindurchtreten eines Werkzeugs zum Schneiden der rekonstituierten Metaphyse des Knochens aufweist, **dadurch gekennzeichnet, dass** der Metaphysenabschnitt (211) einen ersten Schenkel (224), der in Höhe einer Anheftungszone (224A) am Kragen (215) angeheftet ist, sowie einen Sporn (230) umfasst, der aus dem Kragen (215) auf der Außenseite des ersten Schenkels (224) aus einer Anheftungszone (230A) hervorspringt, und dadurch, dass der Kragen (215) zwei Öffnungen (220) umfasst, die sich auf beiden Seiten der Verbindungsachse (D) der Anheftungszonen (224A, 230A) des ersten Schenkels (224) und des Sporns (230) befinden.

4. Prothese nach einem der Ansprüche 1 bis 3, **dadurch gekennzeichnet, dass** jede Öffnung aus mindestens einem länglichen Spalt (20; 220) besteht.

5. Prothese nach Anspruch 4, **dadurch gekennzeichnet, dass** jeder längliche Spalt (20) sich radial bis zur Seite (8C) des Kragens (8) erstreckt.

6. Prothese nach einem der Ansprüche 1 bis 3, **dadurch gekennzeichnet, dass** jede Öffnung aus einer Reihe von Öffnungen mit kleinen Abmessungen, die insbesondere kreisförmig sind, besteht.

## Claims

1. Prosthesis (2) intended to be anchored in a long bone, of the type comprising a stem (4) adapted to as to be inserted in the medullary canal of the said bone, and extended by a metaphysary part (6) connecting, in the region of a connecting zone (10A, 12A) to a flange (8) resting on the metaphysis of the said bone, the metaphysary part (6) comprising a first branch (10) connecting to the flange (8) in the region of a first connecting zone (10A) as well as a second branch (12) connecting to the flange (8) in the region of a second connecting zone (12A), these two branches (10,12) defining a recess (14) within which the initially splintered bone fragments of the metaphysis of the bone can be fixed together, the said flange including two slots (20) for the passage of a tool for cutting the reconstituted metaphysis of the bone, arranged either side of the junction axis (D) of the connecting zones (10A,12A) of the two branches (10,12).

2. Prosthesis according to claim 1, **characterised in that** the said two slots (20) extend at least from the vicinity of the connecting zone (10A) between the first branch (10) and the flange (8), to the vicinity of the connecting zone (12A) between the second branch (12) and the flange (8).

3. Prosthesis (202) designed to be anchored in a long bone, of the type comprising a stem (210) adapted so as to be inserted in the medullary canal of the said bone, and extended by a metaphysary part (211) connecting, in the region of a connecting zone (224A, 230A) to a flange (215) resting on the metaphysis of the said bone, the said flange including at least one opening (220) for the passage of a tool for cutting the reconstituted metaphysis of the bone, **characterised in that** the metaphysary part (211) includes a first branch (224) connecting to the flange (215) in the region of a connecting zone (224A) as well as a lug (230) projecting from the said flange (215) outside the first branch (224) from a connecting zone (230A), and **in that** the said flange (215) includes two openings (220) arranged either side of the junction axis (D) of the connecting zones (224A, 230A) of the first branch (224) and of the said lug (230).

4. Prosthesis according to one of claims 1 to 3, **characterised in that** each opening consists of at least one oblong slot (20 ; 220).

5. Prosthesis according to claim 4, **characterised in that** each oblong slot (20) extends radially to one side (8C) of the flange (8).

6. Prosthesis according to one of claims 1 to 3, **characterised in that** each opening consists of a series of orifices, in particular circular orifices, of small dimensions.
